# EUROPEAN PATENT APPLICATION

(11) **EP 2 319 538 A2**
(43) Date of publication of application: **11.05.2011**
(21) Application number: 10010079.1
(22) Date of filing: 31.05.2005
(51) Int. Cl.: A61K 45/00, A61K 31/439, A61K 31/167, A61K 31/137, A61P 11/00, A61P 11/06, A61P 11/08

(54) **Combinations combrising antimuscarinic agents and corticosteroids**

(30) Priority: 31.05.2004 ES 200401312
(62) Divisional of application: 08014859.6
(71) Applicant: Almirall S.A., 08022 Barcelona (ES)
(72) Inventor: Gras Escardo, Jordi, 08018 Barcelona (ES); Ryder, Hamish, Cambridge CB2 8DZ (GB); Orviz Diaz, Pio, 08980 Sant Feliu de Llobregat Barcelona (ES); Llenas Calvo, Jesus, 08021 Barcelona (ES)
(74) Representative: Srinivasan, Ravi Chandran

(57) **Abstract**

A combination which comprises (a) a corticosteroid and (b) an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid.

## Description

This application claims priority from Spanish patent application number P200401312 filed 31 May 2004, which is incorporated by reference.

The present invention relates to new combinations of certain antimuscarinic agents with corticosteroids and their use in the treatment of respiratory disorders.

### BACKGROUND OF THE INVENTION

Corticosteroids and antimuscarinic agents, in particular antagonists of M3 muscarinic receptors, are two classes of drugs useful in the treatment of respiratory disorders, such as asthma or Chronic Obstructive Pulmonary Diseases (COPD).

Although corticosteroids and antimuscarinic agents may be effective therapies, there exists a clinical need for asthma and COPD therapies having potent and selective action and having an advantageous profile of action.

It is known that both classes of drugs can be used in combination. The International Patent Applications WO0178736, WO0178739, WO0178741, WO0178743, WO0236106 and WO0247667 describe some examples of such combinations.

Combinations of drugs in which the active ingredients operate via different physiological pathways are known to be therapeutically useful. Frequently, the therapeutic advantage arises because the combination can achieve a therapeutically useful effect using lower concentrations of each active component. This enables the side-effects of the medication to be minimised. Thus, the combination can be formulated so that each active ingredient is present at a concentration which is subclinical in cells other than the target disease cells. The combination is nevertheless therapeutically effective in target cells which respond to both ingredients.

### DESCRIPTION OF THE INVENTION

Surprisingly, an unexpectedly beneficial therapeutic effect can be observed in the treatment of inflammatory or obstructive diseases of the respiratory tract if an antimuscarinic of formula (I) used with one or more corticosteroids. In view of this effect the pharmaceutical combinations according to the invention can be used in smaller doses than would be the case with the individual compounds used in monotherapy in the usual way, yet retaining a robust activity in the respiratory tract.

The present invention accordingly provides a combination which comprises (a) a corticosteroid and (b) an antagonist of M3 muscarinic receptors of formula (I) 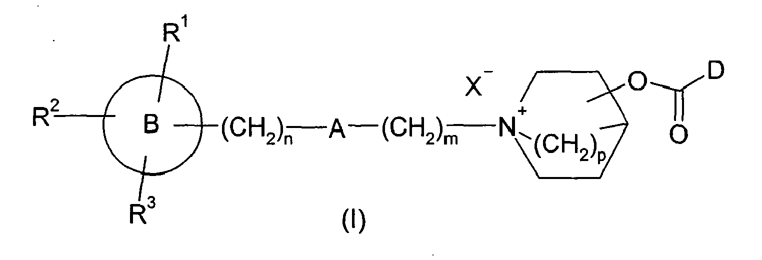 wherein:
B is a phenyl ring, a 5 to 10 membered heteroaromatic group containing one or more heteroatoms or a naphthalenyl, 5,6,7,8-tetrahydronaphthalenyl, benzo[1,3] dioxolyl or biphenyl group;
R¹, R² and R³ each independently represent a hydrogen atom or halogen atom, or a hydroxy group, or a phenyl, -OR⁴, -SR⁴, -NR⁴R⁵, -NHCOR⁴, -CONR⁴R⁵,-CN, -NO₂, -COOR⁴ or -CF₃ group, or a straight or branched lower alkyl group which may optionally be substituted, for example, with a hydroxy or alkoxy group, wherein R⁴ and R⁵ each independently represent a hydrogen atom, straight or branched lower alkyl group or together form an alicyclic ring ; or R¹ and R² together form an aromatic, alicyclic or heterocyclic ring,
n is an integer from 0 to 4;
A represents a -CH₂-,-CH=CR⁶-, -CR⁶=CH-, -CR⁶R⁷-, -CO-, -O-, -S-, -S(O)-, - SO₂- or -NR⁶- group, wherein R⁶ and R⁷ each independently represent a hydrogen atom, straight or branched lower alkyl group or R⁶ and R⁷ together form an alicyclic ring;
m is an integer from 0 to 8 provided that when m = 0, A is not -CH₂-;
p is an integer from 1 to 2 and the substitution in the azoniabicyclic ring may be in the 2, 3 or 4 position including all possible configurations of the asymmetric carbons ;
D represents a group of formula i) or ii): 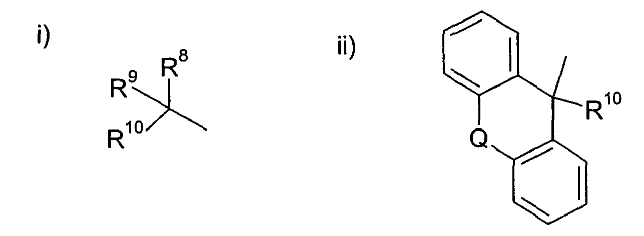 wherein R¹⁰ represents a hydrogen atom, a hydroxy or methyl group or a - CH₂OH group;
R⁸ represents 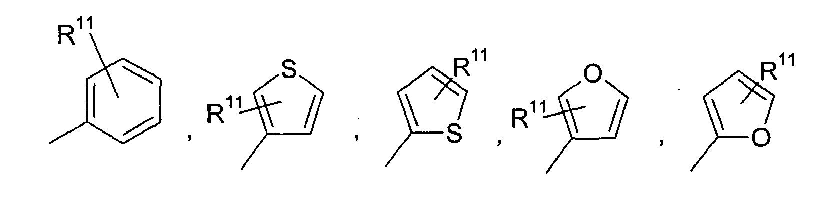
R⁹ represents an alkyl group of 1 to 7 carbon atoms, an alkenyl group containing 2 to 7 carbon atoms, an alkynyl group containing 2 to 7 carbon atoms, a cycloalkyl group of 3 to 7 carbon atoms, or a group selected from: 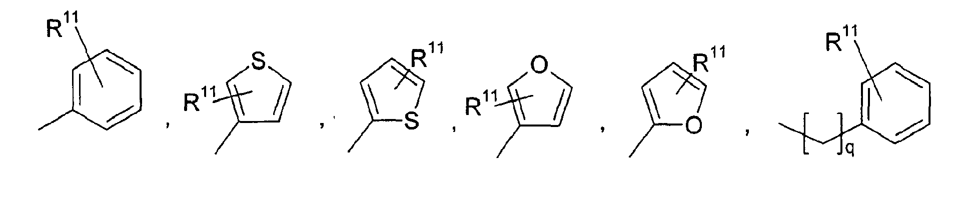 wherein R¹¹ represents a hydrogen or halogen atom, a straight or branched substituted or unsubstituted lower alkyl group, a hydroxy group, an alkoxy group, a nitro group, a cyano group, -CO₂R¹², -NR¹²R¹³ wherein R¹² and R¹³ are identical or different and are selected from hydrogen and straight or branched lower alkyl groups
and Q represents a single bond, -CH₂-, -CH₂-CH₂-, -O-, -O-CH₂-, -S-, -S-CH₂- or -CH=CH-; and
X represents a pharmaceutically acceptable anion of a mono or polyvalent acid optionally in the form of their racemates, their enantiomers, their diastereomers and mixtures thereof.

The compounds of the present invention represented by the formula (I) described above, which may have one or more asymmetric carbons, include all the possible stereoisomers. The single isomers and mixtures of the isomers fall within the scope of the present invention.

As used herein, an alkyl group is typically a lower alkyl group. A lower alkyl group preferably contains 1 to 8, preferably 1 to 6 and more preferably 1 to 4 carbon atoms. In particular it is preferred that such an alkyl group is represented by a methyl, ethyl, propyl, including i-propyl, or butyl including a n-butyl, sec-butyl and tert-butyl group. An alkyl group containing 1 to 7 carbon atoms as mentioned herein may be a C₁₋₄ alkyl group as mentioned above or a straight or branched pentyl, hexyl or heptyl group.

Alkenyl groups having 2 to 7 carbon atoms mentioned herein are straight or branched groups such as ethenyl, or straight or branched propenyl, butenyl, pentenyl, hexenyl or heptenyl. The double bond may be in any position in the alkenyl group, such as on the terminal bond.

Alkynyl groups having 2 to 7 carbon atoms mentioned herein are straight or branched groups such as ethynyl, propynyl or straight or branched butynyl, pentynyl, hexynyl or heptynyl. The triple bond may be in any position in the alkynyl group, such as on the terminal bond.

Alkoxy groups mentioned herein are typically lower alkoxy groups, that is groups containing from 1 to 6 carbon atoms, preferably from 1 to 4 carbon atoms, the hydrocarbon chain being branched or straight. Preferred alkoxy groups include methoxy, ethoxy, n-propoxy, i-propoxy, n-butoxy, sec-butoxy and t-butoxy.

Alicyclic groups or rings as mentioned herein, unless otherwise specified, typically contain from 3 to 8 carbon atoms, preferably from 3 to 6 carbon atoms. Alicyclic rings of 3 to 6 carbon atoms include cyclopropyl, cyclobutyl, cyclopentyl and cyclohexyl.

The aromatic ring as mentioned herein typically contains from 5 to 14, preferably 5 to 10 carbon atoms. Examples of aromatic groups include cyclopentadienyl, phenyl and naphthalenyl.

A heterocyclic or heteroaromatic group mentioned herein is typically a 5 to 10 membered group, such as a 5, 6 or 7 membered group, containing one or more heteroatoms selected from N, S and O. Typically, 1, 2, 3 or 4 heteroatoms are present, preferably 1 or 2 heteroatoms. A heterocyclic or heteroaromatic group may be a single ring or two or more fused rings wherein at least one ring contains a heteroatom. Examples of heterocyclic groups include piperidyl, pyrrolidyl, piperazinyl, morpholinyl, thiomorpholinyl, pyrrolyl, imidazolyl, imidazolidinyl, pyrazolinyl, indolinyl, isoindolinyl, pyridyl, pyrazinyl, pyrimidinyl, pyridazinyl, indolizinyl, isoindolyl, indolyl, indazolyl, purinyl, quinolizinyl, isoquinolyl, quinolyl, quinoxalinyl, quinazolinyl, cinnolinyl, pteridinyl, quinuclidinyl, triazolyl, pyrazolyl, tetrazolyl and thienyl. Examples of heteroaromatic groups include pyridyl, thienyl, furyl, pyrrolyl, imidazolyl, benzothiazolyl, pyridinyl, pyrazolyl, pyrazinyl, pyrimidinyl, pyridazinyl, indolyl, indazolyl, purinyl, quinolyl, isoquinolyl, phthalazinyl, naphthyridinyl, quinoxalinyl, quinazolinyl, cinnolinyl, triazolyl and pyrazolyl.

As used herein a halogen atom includes a fluorine, chlorine, bromine or iodine atom, typically a fluorine, chlorine or bromine atom.

Examples of pharmaceutically acceptable anions of mono or polyvalent acids are the anions derived from inorganic acids such as hydrochloric acid, hydrobromic acid, sulphuric acid, phosphoric acid or organic acids such as methanosulphonic acid, acetic acid, fumaric acid, succinic acid, lactic acid, citric acid or maleic acid. Furthermore, mixtures of the aforementioned acids can be used.

Preferably, the M3 antagonists according to the present invention are those having formula (I) 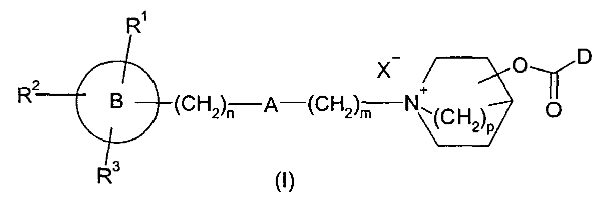 wherein:
- B is a phenyl ring, a C₄ to C₈ heteroaromatic group containing one or more heteroatoms or a naphthalenyl, 5,6,7,8-tetrahydronaphthalenyl or biphenyl group;
- R¹, R² and R³ each independently represent a hydrogen atom or halogen atom, or a hydroxy group, or a phenyl, -OR⁴, -SR⁴, -NR⁴R⁵, -NHCOR⁴, - CONR⁴R⁵, -CN, -NO₂, -COOR⁴ or -CF₃ group, or a straight or branched lower alkyl group which may optionally be substituted, for example, with a hydroxy or alkoxy group, wherein R⁴ and R⁵ each independently represent a hydrogen atom, straight or branched lower alkyl group or together form an alicyclic ring ; or R¹ and R² together form an aromatic, alicyclic or heterocyclic ring,
- n is an integer from 0 to 4;
- A represents a -CH₂-, -CH=CR⁶-, -CR⁶=CH-, -CR⁶R⁷-, -CO-, -O-, -S-, - S(O)-, -SO₂- or -NR⁶- group, wherein R⁶ and R⁷ each independently represent a hydrogen atom, straight or branched lower alkyl group or R⁶ and R⁷ together form an alicyclic ring;
- m is an integer from 0 to 8 provided that when m = 0, A is not -CH₂-;
- p is an integer from 1 to 2 and the substitution in the azoniabicyclic ring may be in the 2, 3 or 4 position including all possible configurations of the asymmetric carbons ;
- D represents a group of formula i) or ii): 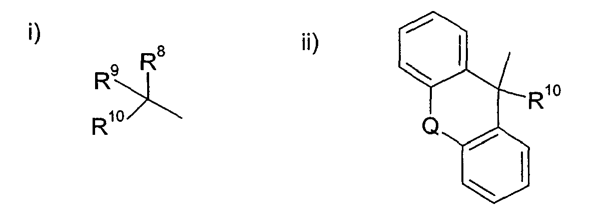 wherein R¹⁰ represents a hydrogen atom, a hydroxy or methyl group; and R⁸ and R⁹ each independently represent 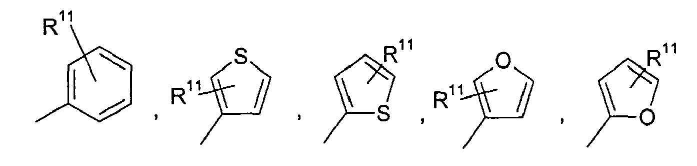 wherein R¹¹ represents a hydrogen or halogen atom or a straight or branched lower alkyl group and Q represents a single bond, -CH₂-, -CH₂-CH₂-, -O-, -O-CH₂-, -S-, -S-CH₂- or -CH=CH-; and
- X represents a pharmaceutically acceptable anion of a mono or polyvalent acid
optionally in the form of their racemates, their enantiomers, their diastereomers and mixtures thereof.

It is a preferred embodiment of the present invention a combination which comprises (a) a corticosteroid and (b) an antagonist of M3 muscarinic receptors of formula (I) 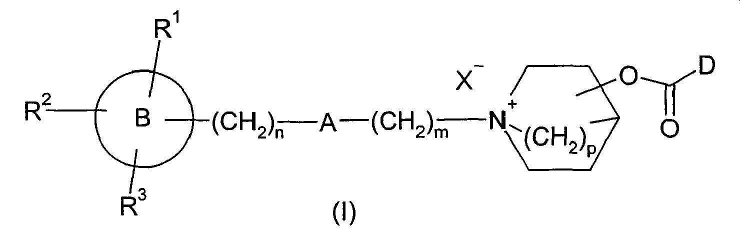 wherein:
B represents a phenyl group;
R¹, R² and R³ represents a hydrogen atom
m is an integer from 1 to 3;
n is zero;
A is a group selected from -O- and -CH₂-;
p is an integer from 1 to 2; the substitution in the azoniabicyclic ring may be in the 2, 3 or 4 position including all possible configurations of the asymmetric carbons ;
-OC(O)D is selected from 2-hydroxy-2,2-dithien-2-ylacetoxy, 9H-xanthene-9-carbonyloxy and (2S)-2-Cyclopentyl-2-hydroxy-2-thien-2-ylacetoxy; and
X represents a pharmaceutically acceptable anion of a mono or polyvalent acid optionally in the form of their racemates, their enantiomers, their diastereomers and mixtures thereof.

The M3 antagonists of the present invention represented by the formula (I) described above, which may have one or more asymmetric carbons, include all the possible stereoisomers. The single isomers and mixtures of the isomers fall within the scope of the present invention.

Those M3 antagonists in which the ester group, -OC(O)D, is attached to the ring comprising the quaternary nitrogen atom at the 3 position are especially preferred.

The M3 antagonists described can optionally be used in the form of their pure enantiomers, mixtures thereof or their racemates. Typically the carbon atom carrying the -OC(O)D group has the (R) configuration.

It is especially preferred that one of 3(R)-(2-hydroxy-2,2-dithien-2-ylacetoxy)-1-(3-phenoxypropyl)-1-azoniabicyclo[2.2.2]octane bromide, (3R)-1-phenethyl-3-(9H-xanthene-9-carbonyloxy)-1-azoniabicyclo[2.2.2]octane bromide and (3R)-3-[(2S)-2-Cyclopentyl-2-hydroxy-2-thien-2-ylacetoxy]-1-(2-phenoxyethyl)-1-azoniabicyclo[2.2.2]octane bromide is used as an M3 antagonist of the invention.

The present invention accordingly provides a combination which comprises (a) a corticosteroid and (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid. Typically the antagonist of M3 muscarinic receptors is 3(R)-(2-hydroxy-2,2-dithien-2-ylacetoxy)-1-(3-phenoxypropyl)-1-azoniabicyclo[2.2.2]octane bromide.

Typically the combination contains the active ingredients (a) and (b) forming part of a single pharmaceutical composition.

For the avoidance of doubt, the formulae depicted above and the term 3(R)-(2-hydroxy-2,2-dithien-2-ylacetoxy)-1-(3-phenoxypropyl)-1-azoniabicyclo[2.2.2]octane is meant to embrace the salts in dissociated, partially dissociated or undissociated form, for example in aqueous solution. The different salts of the compound may exist in the form of solvates, i.e. in the form of hydrates and all these forms are also within the scope of the present invention. Furthermore the different salts and solvates of the compound may exist in amorphous form or in the form of different polymorphs within the scope of the present invention.

Also provided is a product comprising (a) a corticosteroid and (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide), as a combined preparation for simultaneous, separate or sequential use in the treatment of a human or animal patient. Typically the product is for simultaneous, separate or sequential use in the treatment of a respiratory disease which responds to M3 antagonism in a human or animal patient.

The present invention further provides the use of (a) a corticosteroid and (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide), for the preparation of a medicament for simultaneous, concurrent, separate or sequential use in the treatment of a respiratory disease which responds to M3 antagonism in a human or animal patient.

Also provided is the use of (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) for the preparation of a medicament, for simultaneous, concurrent, separate or sequential use in combination with (a) a corticosteroid for the treatment of a respiratory disease which responds to M3 antagonism in a human or animal patient.

Also provided is the use of (a) a corticosteroid for the preparation of a medicament for use in the treatment of a respiratory disease which responds to M3 antagonism in a human or animal patient by simultaneous, concurrent, separate or sequential co-administration with (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide).

The invention also provides the use of (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide), for the preparation of a medicament for use in the treatment of a respiratory disease which responds to M3 antagonism in a human or animal patient by simultaneous, concurrent, separate or sequential co-administration with (a) a corticosteroid.

The present invention further provides a method of treating a human or animal patient suffering from or susceptible to a respiratory disease which responds to M3 antagonism which method comprises simultaneously, concurrently, separately or sequentially administering to said patient an effective amount of (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and (a) a corticosteroid.

Typically said respiratory disease is asthma, acute or chronic bronchitis, emphysema, chronic obstructive pulmonary disease (COPD), bronchial hyperreactivity or rhinitis, in particular asthma or chronic obstructive pulmonary disease (COPD).

Preferably said patient is human.

Also provided is a pharmaceutical composition comprising (a) a corticosteroid; and (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide), in association with (c) a pharmaceutically acceptable carrier or diluent.

The invention also provides a kit of parts comprising (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) together with instructions for simultaneous, concurrent, separate or sequential use in combination with (a) a corticosteroid for the treatment of a human or animal patient suffering from or susceptible to a respiratory disease which responds to M3 antagonism.

Further provided is a package comprising (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and (a) a corticosteroid for the simultaneous, concurrent, separate or sequential use in the treatment of a respiratory disease which responds to M3 antagonism.

Further provided is a combination, product, kit of parts or package as hereinabove described wherein such combination, product, kit of parts or package further comprises (c) another active compound selected from: (a) PDE IV inhibitors, (b) β2 agonists, (c) leukotriene D4 antagonists, (d) inhibitors of egfr-kinase, (e) p38 kinase inhibitors and (f) NK1 receptor agonists for simultaneous, separate or sequential use. Typically the additional active compound (c) is selected from the group consisting of (a) PDE IV inhibitors and (b) β2 agonists.

It is a embodiment of the present invention that the combination, product, kit of parts or package comprise (b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and (a) a corticosteroid as the sole active compounds.

It is also an embodiment of the present invention the use of b) an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and (a) a corticosteroid without any other active compound for the preparation of a medicament for simultaneous, concurrent, separate or sequential use in the treatment of a respiratory disease which responds to M3 antagonism in a human or animal patient.

The preferred corticosteroids to be used in the combinations of the invention are prednisolone, methylprednisolone, dexamethasone, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, deprodone propionate, fluticasone propionate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Particularly preferred corticosteroids under the present invention are: dexamethasone, budesonide, beclomethasone, triamcinolone, dexamethasone, mometasone, ciclesonide, fluticasone, flunisolide, dexamethasone sodium phosphate and esters thereof as well as 6α,9α -difluoro-17α -[(2-furanylcarbonyl)oxy]-11β-hydroxy-16α-methyl-3-oxoandrosta-1,4-diene-17β-carbothioic acid (S)-fluoromethyl ester.

Still more preferred corticosteroids under the present invention are: budesonide, beclomethasone dipropionate, mometasone furoate, ciclesonide, triamcinolone, triamcinolone acetonide, triamcinolone hexaacetonide and fluticasone propionate optionally in the form of their racemates, their enantiomers, their diastereomers and mixtures thereof, and optionally their pharmacologically-compatible acid addition salts. Even more preferred are budesonide, beclomethasone dipropionate, mometasone furoate, ciclesonide and fluticasone propionate. The most preferred corticosteroids of the present invention are budesonide and beclomethasone dipropionate.

Any reference to corticosteroids within the scope of the present invention includes a reference to salts or derivatives thereof which may be formed from the corticosteroids. Examples of possible salts or derivatives include: sodium salts, sulphobenzoates, phosphates, isonicotinates, acetates, propionates, dihydrogen phosphates, palmitates, pivaiates, farnesylates, aceponates, suleptanates, prednicarbates, furoates or acetonides. In some cases the corticosteroids may also occur in the form of their hydrates.

A preferred embodiment of the present invention is a combination of an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) with a corticosteroid selected from budesonide, beclomethasone dipropionate, mometasone furoate, ciclesonide and fluticasone propionate.

A particularly preferred embodiment of the present invention is a combination of an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) with a corticosteroid selected from budesonide, beclomethasone dipropionate, mometasone furoate, ciclesonide and fluticasone propionate.

Another embodiment of the present invention is a combination of an M3 antagonist selected from the group consisting of 3(R)-(2-hydroxy-2,2-dithien-2-ylacetoxy)-1-(3-phenoxypropyl)-1-azoniabicyclo[2.2.2]octane bromide, (3R)-1-phenethyl-3-(9H-xanthene-9-carbonyloxy)-1-azoniabicyclo[2.2.2]octane bromide, and (3R)-3-[(2S)-2-Cyclopentyl-2-hydroxy-2-thien-2-ylacetoxy]-1-(2-phenoxyethyl)-1-azoniabicyclo[2.2.2]octane bromide with a corticosteroid selected from budesonide, beclomethasone dipropionate, mometasone furoate, ciclesonide and fluticasone propionate.

According to one embodiment of the invention the antagonist of M3 muscarinic receptors is a compound of formula (I) and in particular 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and the corticosteroid is a beclomethasone derivative, in particular beclomethasone dipropionate.

According to another embodiment of the invention the antagonist of M3 muscarinic receptors is a compound of formula (I) and in particular 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and the corticosteroid is budesonide.

The combinations of the invention can optionally comprise one or more additional active substances which are known to be useful in the treatment of respiratory disorders, such as PDE4 inhibitors, β2 agonists or glucocorticoids, leukotriene D4 inhibitors, inhibitors of egfr-kinase, p38 kinase inhibitors and/or NK1-receptor antagonists.

Examples of suitable PDE4 inhibitors that can be combined with M3-antagonists and corticosteroids are denbufylline, rolipram, cipamfylline, arofylline, filaminast, piclamilast, mesopram, drotaverine hydrochloride, lirimilast, roflumilast, cilomilast, 6-[2-(3,4-Diethoxyphenyl)thiazol-4-yl]pyridine-2-carboxylic acid, (R)-(+)-4-[2-(3-Cyclopentyloxy-4-methoxyphenyl)-2-phenylethyl]pyridine, N-(3,5-Dichloro-4-pyridinyl)-2-[1-(4-fluorobenzyl)-5-hydroxy-1H-indol-3-yl]-2-oxoacetamide, 9-(2-Fluorobenzyl)-N6-methyl-2-(trifluoromethyl)adenine, N-(3,5-Dichloro-4-pyridinyl)-8-methoxyquinoline-5-carboxamide, N-[9-Methyl-4-oxo-1-phenyl-3,4,6,7-tetrahydropyrrolo[3,2,1-jk][1,4]benzodiazepin-3(R)-yl]pyridine-4-carboxamide, 3-[3-(Cyclopentyloxy)-4-methoxybenzyl]-6-(ethylamino)-8-isopropyl-3H-purine hydrochloride, 4-[6,7-Diethoxy-2,3-bis(hydroxymethyl)naphthalen-1-yl]-1-(2-methoxyethyl)pyridin-2(1H)-one, 2-carbomethoxy-4-cyano-4-(3-cyclopropylmethoxy-4-difluroromethoxyphenyl)cyclohexan1-one, *cis* [4-cyano-4-(3-cyclopropylmethoxy-4-difluoromethoxyphenyl)cyclohexan-1-ol, ONO-6126 (Eur Respir J 2003, 22(Suppl. 45): Abst 2557) and the compounds claimed in the PCT patent applications number WO03/097613 and PCT/EP03/14722 and in the Spanish patent application number P200302613.

Examples of suitable β2-agonists that can be combined with M3-antagonists and corticosteroids are: arformoterol, bambuterol, bitolterol, broxaterol, carbuterol, clenbuterol, dopexamine, fenoterol, formoterol, hexoprenaline, ibuterol, isoetharine, isoprenaline, levosalbutamol, mabuterol, meluadrine, metaprotenerol, nolomirole, orciprenaline, pirbuterol, procaterol, reproterol, ritodrine, rimoterol, salbutamol, salmefamol, salmeterol, sibenadet, sotenerot, sulfonterol, terbutaline, tiaramide, tulobuterol, GSK-597901, GSK-159797, GSK-678007, GSK-642444, GSK-159802, HOKU-81, (-)-2-[7(S)-[2(R)-Hydroxy-2-(4-hydroxyphenyl)ethylamino]-5,6,7,8-tetrahydro-2-naphthyloxy]-N,N-dimethylacetamide hydrochloride monohydrate, carmoterol, QAB-149 and 5-[2-(5,6-diethylindan-2-ylamino)-1-hydroxyethyl]-8-hydroxy-1H-quinolin-2-one, 4-hydroxy-7-[2-{[2-{[3-(2-phenylethoxy)propyl]sulfonyl}ethyl]amino}ethyl]-2(3H)-benzothiazolone, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tert-butylamino)ethanol and 1-(4-ethoxycarbonylamino-3-cyano-5-fluorophenyl)-2-(tert-butylamino)ethanol optionally in the form of their racemates, their enantiomers, their diastereomers, and mixtures thereof, and optionally their pharmacologically-compatible acid addition salts.

Examples of suitable LTD4 antagonists that can be combined with M3 antagonists and corticosteroids are tomelukast, lbudilast, pobilukast, pranlukast hydrate, zafirlukast, ritolukast, verlukast, sulukast, cinalukast, iralukast sodium, montelukast sodium, 4-[4-[3-(4-Acetyl-3-hydroxy-2-propylphenoxy)propylsulfonyl]phenyl]-4-oxobutyric acid, [[5-[[3-(4-Acetyl-3-hydroxy-2-propylphenoxy)propyl]thio]-1,3,4-thiadiazol-2-yl]thio]acetic acid, 9-[(4-Acetyl-3-hydroxy-2-n-propylphenoxy)methyl]-3-(1H-tetrazol-5-yl)-4H-pyrido[1,2-a]pyrimidin-4-one, 5-[3-[2-(7-Chloroquinolin-2-yl)vinyl]phenyl]-8-(N,N-dimethylcarbamoyl)-4,6-dithiaoctanoic acid sodium salt; 3-[1-[3-[2-(7-Chloroquinolin-2-yl)vinyl]phenyl]-1-[3-(dimethylamino)-3-oxopropylsulfanyl]methylsulfanyl]propionic acid sodium salt, 6-(2-Cyclohexylethyl)-[1,3,4]thiadiazolo[3,2-a]-1,2,3-triazolo[4,5-d]pyrimidin-9(1H)-one, 4-[6-Acetyl-3-[3-(4-acetyl-3-hydroxy-2-propylphenylthio)propoxy]-2-propylphenoxy]butyric acid, (R)-3-Methoxy-4-[1-methyl-5-[N-(2-methyl-4,4,4-trifluorobutyl)carbamoyl]indol-3-ylmethyl]-N-(2-methylphenylsulfonyl)benzamide, (R)-3-[2-Methoxy-4-[N-(2-methylphenylsulfonyl)carbamoyl]benzyl]-1-methyl-N-(4,4,4-trifluoro-2-methylbutyl)indole-5-carboxamide, (+)-4(S)-(4-Carboxyphenylthio)-7-[4-(4-phenoxybutoxy)phenyl]-5(Z)-heptenoic acid and the compounds claimed in the PCT patent application number PCT/EP03/12581.

Examples of suitable inhibitors of egfr-kinase that can be combined with M3 antagonists and corticosteroids are palifermin, cetuximab, gefitinib, repifermin, erlotinib hydrochloride, canertinib dihydrochloride, lapatinib, and N-[4-(3-Chloro-4-fluorophenylamino)-3-cyano-7-ethoxyquinolin-6-yl]-4-(dimethylamino)-2(E)-butenamide.

Examples of suitable p38 kinase inhibitors that can be combined with M3 antagonists and corticosteroids are chlormethiazole edisylate, doramapimod, 5-(2,6-Dichlorophenyl)-2-(2,4-difluorophenylsulfanyl)-6H-pyrimido[3,4-b]pyridazin-6-one, 4-Acetamido-N-(tert-butyl)benzamide, SCIO-469 (described in Clin Pharmacol Ther 2004, 75(2): Abst PII-7 and VX-702 described in Circulation 2003, 108(17, Suppl. 4): Abst 882.

Examples of suitable NK1-receptor antagonists that can be combined with M3 antagonists and corticosteroids are nolpitantium besilate, dapitant, lanepitant, vofopitant hydrochloride, aprepitant, ezlopitant, N-[3-(2-Pentylphenyl)propionyl]-threonyl-N-methyl-2,3-dehydrotyrosyl-leucyl-D-phenylalanyl-allo-threonyl-asparaginyl-serine C-1.7-O-3.1 lactone, 1-Methylindol-3-ylcarbonyl-[4(R)-hydroxy]-L-prolyl-[3-(2-naphthyl)]-L-alanine N-benzyl-N-methylamide, (+)-(2S,3S)-3-[2-Methoxy-5-(trifluoromethoxy)benzylamino]-2-phenylpiperidine, (2R,4S)-N-[1-[3,5-Bis(trifluoromethyl)benzoyl]-2-(4-chlorobenzyl)piperidin-4-yl]quinoline-4-carboxamide, 3-[2(R)-[1 (R)-[3,5-Bis(trifluoromethyl)phenyl]ethoxy]-3(S)-(4-fluorophenyl)morpholin-4-ylmethyl]-5-oxo-4,5-dihydro-1H-1,2,4-triazole-1-phosphinic acid bis(N-methyl-D-glucamine) salt; [3-[2(R)-[1(R)-[3,5-bis(trifluoromethyl)phenyl]ethoxy]-3(S)-(4-fluorophenyl)-4-morpholinylmethyl]-2,5-dihydro-5-oxo-1H-1,2,4-triazol-1-yl]phosphonic acid 1-deoxy-1-(methylamino)-D-glucitol (1:2) salt, 1'-[2-[2(R)-(3,4-Dichlorophenyl)-4-(3,4,5-trimethoxybenzoyl)morpholin-2-yl]ethyl]spiro[benzo[c]thiophen-1 (3H)-4'-piperidine] 2(S)-oxide hydrochloride and the compound CS-003 described in Eur Respir J 2003, 22(Suppl. 45): Abst P2664.

The combinations of the invention may be used in the treatment of any disorder which is susceptible to amelioration by simultaneous, concomitant or sequential antagonism of M3 muscarinic receptors and corticosteroids. Thus, the present application encompasses methods of treatment of these disorders, as well as the use of the combinations of the invention in the manufacture of a medicament for the treatment of these disorders.

Preferred examples of such disorders are those respiratory diseases, wherein the use of bronchodilating agents is expected to have a beneficial effect, for example asthma, acute or chronic bronchitis, emphysema, or Chronic Obstructive Pulmonary Disease (COPD).

The active compounds in the combination, i.e. the M3 antagonist of the invention, the corticosteroid and any other optional active compounds may be administered together in the same pharmaceutical composition or in different compositions intended for separate, simultaneous, concomitant or sequential administration by the same or a different route.

In one embodiment the present invention provides a kit of parts comprising an antagonist of M3 muscarinic receptors of formula (I) together with instructions for simultaneous, concurrent, separate or sequential use in combination with a corticosteroid for the treatment of a respiratory disease which responds to M3 antagonism.

In a preferred embodiment the present invention provides a kit of parts comprising an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) together with instructions for simultaneous, concurrent, separate or sequential use in combination with a corticosteroid for the treatment of a respiratory disease which responds to M3 antagonism.

In another embodiment the present invention provides a package comprising an antagonist of M3 muscarinic receptors of formula (I) and a corticosteroid for the simultaneous, concurrent, separate or sequential use in the treatment of a respiratory disease which responds to M3 antagonism.

In another embodiment the present invention consists of a package comprising an antagonist of M3 muscarinic receptors of formula (I) and in particular an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid (in particular 3(R)-(2-hydroxy-2,2-dithien-2-ylacetoxy)-1-(3-phenoxypropyl)-1-azoniabicyclo[2.2.2]octane bromide) and a corticosteroid for the simultaneous, concurrent, separate or sequential use in the treatment of a respiratory disease which responds to M3 antagonism.

In a preferred embodiment of the invention the active compounds in the combination are administered by inhalation through a common delivery device, wherein they can be formulated in the same or in different pharmaceutical compositions.

In the most preferred embodiment the M3 antagonist of the invention and the corticosteroid are both present in the same pharmaceutical composition and are administered by inhalation through a common delivery device.

In one aspect the invention provides a combination as herein defined **characterised in that** the active ingredients (a) and (b) form part of a single pharmaceutical composition.

In another aspect the invention provides a process for the production of a pharmaceutical composition as herein defined **characterised in that** an antagonist of M3 muscarinic receptors, a corticosteroid and optionally other additives and/or carriers are mixed and processed by methods known per se.

The active compounds in the combination, i.e. the M3 antagonist of the invention, the corticosteroid and any other optional active compounds may be administered by any suitable route, depending on the nature of the disorder to be treated, e.g. orally (as syrups, tablets, capsules, lozenges, controlled-release preparations, fast-dissolving preparations, lozenges, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, natural, synthetic or semisynthetic oils such as peanut oil and olive oil, glycerine or water with flavouring, sweetener and/or colouring agent.

Where the composition is in the form of a tablet, any pharmaceutical carrier routinely used for preparing solid formulations may be used. Examples of such carriers include celluloses, stearates such as magnesium stearate or stearic acid, talc, gelatine, acacia, starches, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with binders, lubricants, inert diluents, lubricating, surface active or dispersing agents. Moulded tablets may be made by moulding in a suitable machine a mixture of the powdered blend comprising the active compounds moistened with an inert liquid diluent and optionally dried and sieved. The tablets may optionally be coated or scored and may be formulated so as to provide modified (i.e. slow or controlled) release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in different primary packaging systems (such as capsules and cartridges of for example gelatine or blisters of for example laminated aluminium foil), for use in an inhaler or insufflator.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

Formulations generally contain a powder mix for inhalation of the compounds of the invention and a suitable powder base (carrier substance) such as lactose or starch. Use of lactose is preferred. Each capsule or cartridge may generally contain between 2µg and 400 µg of each therapeutically active ingredient. Alternatively, the active ingredient (s) may be presented without excipients.

For single dose inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients. Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring slides (e. g.US 5201308 and WO 97/00703) or measuring plungers with a local or circumferential recess to displace a certain volume of powder from the container to a delivery chamber or an air conduit e. g. EP 0505321, WO 92/04068 and WO 92/04928.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi- dose devices, powder adhesion onto the inner walls of the air conduits and the de- agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even stricter.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with.

Such atomisers are described, for example, in PCT Patent Application No. WO 91/14468 and International Patent Application No. WO 97/12687, reference here being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogencontaining chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant. The aerosol composition may be free from excipients other than the propellant or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg by micronisation or supercritical fluid techniques. The desired fraction may be separated out by air classification or sieving. Preferably, the particles will be crystalline.

Achieving a high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose, manitol or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, mucoadhesive agents, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

The proportions in which (a) the corticosteroid and (b) the antagonist of M3 muscarinic receptors may be used according to the invention are variable. Active substances (a) and (b) may possibly be present in the form of their solvates or hydrates. Depending on the choice of the compounds (a) and (b), the weight ratios which may be used within the scope of the present invention vary on the basis of the different molecular weights of the various salt forms. The pharmaceutical combinations according to the invention may contain (a) and (b) generally in a ratio by weight (b):(a) ranging from 1:100 to 100: 1, preferably from 1:50 to 50:1.

The weight ratios specified below are based on the compound (b) expressed as 3(R)-(2-hydroxy-2,2-dithien-2-ylacetoxy)-1-(3-phenoxypropyl)-1-azoniabicyclo[2.2.2]octane bromide and the corticosteroids budesonide and beclomethasone dipropionate which are particularly preferred according to the invention.

The pharmaceutical combinations according to the invention may contain (a) and (b) in the case of budesonide, for example, in a ratio by weight (b):(a) ranging from 1:10 to 50:10, preferably from 1:5 to 10:1, preferably from 1:4 to 5:1, most preferably from 1:2 to 2:1.

The pharmaceutical compositions according to the invention containing the combinations of (a) and (b) are normally administered so that 3(R)-(2-hydroxy-2,2-dithien-2-ylacetoxy)-1-(3-phenoxypropyl)-1-azoniabicyclo[2.2.2]octane bromide and budesonide are present together in doses of 5 to 5000 µg, preferably from 10 to 2000 µg, more preferably from 15 to 1000 µg, better still from 20 to 800 µg per single dose.

For example, without restricting the scope of the invention thereto, combinations in which 3(R)-(2-hydroxy-2,2-dithien-2-ylacetoxy)-1-(3-phenoxypropyl)-1-azoniabicyclo[2.2.2]octane bromide is used as (b) and budesonide is used as (a), the compositions according to the invention may contain for instance from 20 to 1000 µg of 3(R)-(2-hydroxy-2,2-dithien-2-ylacetoxy)-1-(3-phenoxypropyl)-1-azoniabicyclo[2.2.2]octane bromide and from 50 to 500 µg of budesonide.

For example, the active substance combinations according to the invention may contain 3(R)-(2-hydroxy-2,2-dithien-2-ylacetoxy)-1-(3-phenoxypropyl)-1-azoniabicyclo[2.2.2]octane bromide and (a) in the case of beclomethasone dipropionate, in a ratio by weight (b):(a) in the range from about 1:100 to 50:1, preferably 1:50 to 30:1, preferably 1:10 to 20:1, most preferably from 1:5 to 10: 1.

The pharmaceutical compositions according to the invention containing the combinations of (a) and (b) are usually administered so that 3(R)-(2-hydroxy-2,2-dithien-2-ylacetoxy)-1-(3-phenoxypropyl)-1-azoniabicyclo[2.2.2]octane bromide and beclomethasone dipropionate are present together in dosages of 5 to 5000 µg, preferably from 50 to 2000µg, more preferably from 100 to 1000µg, even more preferably from 200 to 800µg per single dose.

For example, without restricting the scope of the invention thereto, combinations in which 3(R)-(2-hydroxy-2,2-dithien-2-ylacetoxy)-1-(3-phenoxypropyl)-1-azoniabicyclo[2.2.2]octane bromide is used as (b) and beclomethasone dipropionate is used as (a), the compositions according to the invention may contain for instance from 20 to 1000 µg of 3(R)-(2-hydroxy-2,2-dithien-2-ylacetoxy)-1-(3-phenoxypropyl)-1-azoniabicyclo[2.2.2]octane bromide and from 20 to 800 µg of beclomethasone dipropionate.

The aforementioned examples of possible doses applicable for the combinations according to the invention are to be understood as referring to doses per single application. However, these examples are not be understood as excluding the possibility of administering the combinations according to the invention multiple times. Depending on the medical need patients may receive also multiple inhalative applications. As an example patients may receive the combinations according to the invention for instance two or three times (e. g. two or three puffs with a powder inhaler, an MDI etc) in the morning of each treatment day. As the aforementioned dose examples are only to be understood as dose examples per single application (i. e. per puff) multiple application of the combinations according to the invention leads to multiple doses of the aforementioned examples. The application of the combositions according to the invention can be for instance once a day, or depending on the duration of action of the anticholinergic agent twice a day, or once every 2 or 3 days, or even on an "as needed" basis (three or more times a day on occasional days).

Preferably the composition is in unit dosage form, for example a tablet, capsule or metered aerosol dose, so that the patient may administer a single dose.

Each dosage unit contains suitably from 20 µg to 1000 µg and preferably from 50 µg to 400 µg of an M3 antagonist according to the invention or a pharmaceutical acceptable salt thereof and 1 µg to 800 µg, and preferably from 20 µg to 500 µg of a corticosteroid according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day.

It is contemplated that all active agents would be administered at the same time, or very close in time. Alternatively, one or two actives could be taken in the morning and the other(s) later in the day. Or in another scenario, one or two actives could be taken twice daily and the other(s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

The following preparations forms are cited as formulation examples:

### Example 1 Inhalable powder

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Budesonide | 200 |
| Lactose | 10.200 |

### Example 2 Inhalable powder

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Beclomethasone propionate | 125 |
| Lactose | 10.275 |

### Example 3 Inhalable powder

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Fluticasone propionate | 125 |
| Lactose | 10.275 |

### Example 4 Inhalable powder

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Mometasone furoate | 250 |
| Lactose | 10.150 |

### Example 5 Inhalable powder

| Ingredient | Amount in µg |
|---|---|
| 3(R)-(2-hydroxy-2,2-dithien-2-ylacetoxy)-1-(3-phenoxypropyl)-1-azoniabicyclo[2.2.2]octane bromide | 100 |
| Ciclesonide | 250 |
| Lactose | 10.150 |

### Example 6 Aerosol

| Ingredient | % by weight |
|---|---|
| 3(R)-(2-hydroxy-2,2-dithien-2-ylacetoxy)-1-(3-phenoxypropyl)-1-azoniabicyclo[2.2.2]octane bromide | 0,33 |
| Budesonide | 0,55 |
| Lecithin | 0,27 |
| TG134a:TG227 2:3 | ad 100 |

### Example 7 Aerosol

| Ingredient | % by weight |
|---|---|
| 3(R)-(2-hydroxy-2,2-dithien-2-ylacetoxy)-1-(3-phenoxypropyl)-1-azoniabicyclo[2.2.2]octane bromide | 0,25 |
| Fluticasone propionate | 0,40 |
| Isopropyl myristate | 0,10 |
| TG 227 | ad 100 |

### Pharmacological activity

Surprisingly, an unexpectedly beneficial therapeutic effect can be observed in the treatment of inflammatory or obstructive diseases of the respiratory tract if an antimuscarinic of formula (I) used with one or more corticosteroids. In view of this effect the pharmaceutical combinations according to the invention can be used in smaller doses than would be the case with the individual compounds used in monotherapy in the usual way. This reduces unwanted side effects such as may occur when corticosteroids are administered, for example.

The compositions above are specific examples of preferred embodiments of the invention, wherein an M3 antagonist of Formula I is combined with a corticosteroid. These new combinations present significant therapeutic advantages with respect to the combinations of M3 antagonists and a corticosteroid already known in the art.

In particular, the combination of an M3 antagonist of Formula I with a corticosteroid such as budesonide or beclomethasone, produces significantly and consistently more inhibition of the contractile response of the tracheal ring to allergens than a therapeutically equivalent combination of tiotropium bromide with budesonide or beclomethasone.

The following comparative examples describe the advantageous properties of combinations comprising the most preferred M3 antagonist of the invention , i.e. 3(R)-(2-hydroxy-2,2-dithien-2-ylacetoxy)-1-(3-phenoxypropyl)-1-azoniabicyclo[2.2.2]octane bromide (compound 1).

### Material and Methods

Male Dunkin-Hartley guinea-pigs (weighing 380-420 g) from Harlan Ibérica (St. Feliu de Codines, Spain) are used. They are housed, with free access to food and water, in rooms kept at 22±2 °C under a 12 hours light-dark cycle until the start of the experiment.

The animals are sensitised by means of two sessions of aerosolization with a solution of 5 mg/ml of ovoalbumin on days zero and 7 of the study. The aerosolization procedure consists of two 30-s nebulisations (Efbe air brush apparatus) with an interval of 5 minutes with animals maintained in a plexiglass box for 10 minutes since the beginning of the procedure.

Between days 14 to 20 from the beginning of the experiment the animals are euthanised and the tracheal tissue is removed. A single tracheal ring is excised and suspended in an organ bath containing Krebs solution. Once attached to a force isometric transducer, it is submitted to a basal resting tension of 1 g, equilibrated with a mixture a 5 % CO₂ in O₂ and maintained at 37° C.

The preparations are allowed to equilibrate for a period of not less than 60 minutes and then the vehicle or the compound(s) to be tested are added to the bath. The corticosteroids (if present) are added first, and, after an incubation period of 45 minutes, the M3 antagonist are subsequently added allowing the system to stand for another 15 minutes. At this moment ovoalbumin is added (at a final concentration in the bath of 10 µg/ml) to elicit the contractile response which is measured immediately.

The contractile responses measured with a force isometric transducer are expressed in mg.

### Results

The results obtained are shown in Table 1.

**TABLE 1.- COMPARATIVE EFFECTS OF BUDESONIDE, BECLOMETHASONE AND THEIR COMBINATIONS WITH COMPOUND 1 AND TIOTROPIUM ON THE INHIBITION THE CONTRACTION INDUCED BY THE ANTIGEN IN OVOALBUMIN-SENSITIZED GUINEA-PIG ISOLATED TRACHEAL RINGS**

| COMPOUND | NUMBER OF ANIMALS TESTED | CONTRACTILE RESPONSE TO OVOALBUMIN (in mg) (mean±sem) | % INHIBITION OF THE CONTRACTIL E RESPONSE |
|---|---|---|---|
| VEHICLE | 23 | 886±108 | --- |
| | | | |
| BUDESONIDE (10 µM) | 17 | 435±68 | 51 |
| | | | |
| BECLOMETHASONE (10 µM) | 6 | 778±83 | 12 |
| | | | |
| COMPOUND 1 (10 µM)+ BUDESONIDE (10 µM) | 9 | 325±81 | 63 |
| | | | |
| COMPOUND 1 (10 µM)+ BECLOMETHASONE (10 µM) | 7 | 478±61 | 46 |
| | | | |
| TIOTROPIUM (10 µM)+ BUDESONIDE (10 µM) | 8 | 523±147 | 41 |
| | | | |
| TIOTROPIUM (10 µM)+ BECLOMETHASONE (10 µM) | 6 | 612±109 | 31 |

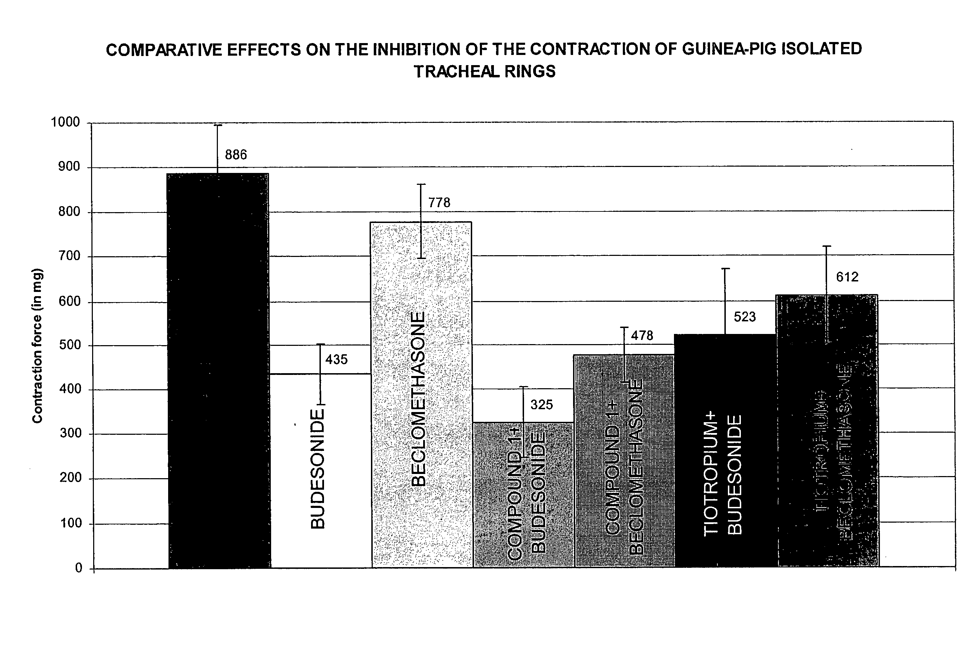

The results summarised in Table 1 and Figure 1 show the following effects:
Budesonide alone produces a consistent effect inhibiting the contractile response whilst beclomethasone produces a smaller effect. The budesonide results agree with those reported by Persson et al. (Int Arch Allergy Appl lmmunol 1989;88:381-385) that concluded that budesonide reduced the sensitivity to antigen-induced IgE-driven contractions in guinea-pig tracheal rings.

When compound 1 is associated with budesonide, but maintaining its respective incubation time, the inhibition is greater than the one obtained by budesonide alone.

On the other hand, when tiotropium is associated with budesonide the inhibition is slightly smaller than the one elicited by the steroidal agent alone.

When compound 1 is associated with beclomethasone the inhibition obtained is greater than the one elicited by beclomethasone alone.

When tiotropium is associated with beclomethasone the inhibition obtained is also greater than the one elicited by the steroidal agent alone but the effect is not as big as the one obtained with compound 1.

In any event, the inhibitory effects elicited by the associations of corticosteroids and compound 1 are greater than those elicited by the associations of the same corticosteroids and tiotropium.

In conclusion, the present experiment suggests that the associations of the M3 antagonist of the present invention with steroidal agents would be more active inhibiting the contractile response to the antigen in actively sensitised guinea pig tracheal ring's than the associations of tiotropium with the same steroidal agents.

Consequently, the combinations of the invention possess therapeutically advantageous properties, which make them particularly suitable for the treatment of respiratory diseases in all kind of patients.

### BRIEF DESCRIPTION OF FIGURES

FIG. 1 shows the inhibitory effect on the contraction induced by the antigen in ovoalbumin-sensitized guinea-pig isolated tracheal ring of corticosteroids alone or in combination with the M3 antagonists of the present invention

## Claims

1. A combination which comprises (a) a corticosteroid and (b) an antagonist of M3 muscarinic receptors which is 3(R)-(2-hydroxy-2,2-dithien-2-ylacetoxy)-1-(3-phenoxypropyl)-1-azoniabicyclo[2.2.2]octane, in the form of a salt having an anion X, which is a pharmaceutically acceptable anion of a mono or polyvalent acid.

2. A combination according to claim 1 wherein the antagonist of M3 muscarinic receptor (b) is 3(R)-(2-hydroxy-2,2-dithien-2-ylacetoxy)-1-(3-phenoxypropyl)-1-azoniabicyclo[2.2.2]octane bromide.

3. A combination according to claim 1 or 2 **characterised in that** the active ingredients (a) and (b) form part of a single pharmaceutical composition.

4. A product comprising (a) corticosteroid and (b) an antagonist of M3 muscarinic receptors as defined in claims 1 or 2, as a combined preparation for simultaneous, concurrent, separate or sequential use in the treatment of a patient suffering from or susceptible to a respiratory disease which responds to M3 antagonism.

5. A kit of parts comprising (b) an antagonist of M3 muscarinic receptors as defined in claim 1 or 2 together with instructions for simultaneous, concurrent, separate or sequential use in combination with (a) a corticosteroid for the treatment of a or animal patient suffering from or susceptible to a respiratory disease which responds to M3 antagonism.

6. A package comprising (b) an antagonist of M3 muscarinic receptors as defined in claim 1 or 2 and (a) a corticosteroid for the simultaneous, concurrent, separate or sequential use in the treatment of a respiratory disease which responds to M3 antagonism.

7. Use of (b) an antagonist of M3 muscarinic receptors as defined in claim 1 or 2 for the preparation of a medicament, for simultaneous, concurrent, separate or sequential use in combination with (a) a corticosteroid for the treatment of a respiratory disease which responds to M3 antagonism in a patient.

8. Use of (a) a corticosteroid for the preparation of a medicament, for simultaneous, concurrent, separate or sequential use in combination with (b) an antagonist of M3 muscarinic receptors as defined in claim 1 or 2 for the treatment of a respiratory disease which responds to M3 antagonism in a patient.

9. Use of (a) a corticosteroid and (b) an antagonist of M3 muscarinic receptors as defined in claim 1 or 2, for the preparation of a medicament for simultaneous, concurrent, separate or sequential use in the treatment of a respiratory disease which responds to M3 antagonism in a human or animal patient.

10. A method of treating a human or animal patient suffering from or susceptible to a respiratory disease or condition which responds to M3 antagonism which method comprises simultaneously, concurrently, separately or sequentially administering to said patient an effective amount of (b) an antagonist of M3 muscarinic receptors as defined in claim 1 or 2 and (a) a corticosteroid.

11. The product according to claim 4, kit of parts according to claim 5, use according to claims 7 to 9 or method according to claim 10 wherein the respiratory disease is asthma or chronic obstructive pulmonary disease (COPD).

12. The combination, product, kit of parts, package, use or method according to anyone of claims 1 to 11 wherein the corticosteroid is selected from the group comprising dexamethasone, budesonide, beclomethasone, triamcinolone, dexamethasone, mometasone, ciclesonide, fluticasone, flunisolide, dexamethasone sodium phosphate and esters thereof as well as 6α,9α -difluoro-17α-[(2-furanylcarbonyl)oxy]-11β-hydroxy-16α-methyl-3-oxoandrosta-1,4-diene-17β-carbothioic acid (S)-fluoromethyl ester.

13. The combination, product, kit of parts, package, use or method according to anyone of claims 1 to 12 wherein the corticosteroid is selected from the group comprising budesonide and beclomethasone dipropionate.

14. The combination, product, kit of parts, package, use or method according to anyone of claims 1 to 13 wherein the corticosteroid is budesonide.

15. The combination, kit of parts, package, use or method according to anyone of claims 1 to 13 wherein the corticosteroid is beclomethasone dipropionate.

16. The combination according to claims 1 to 3, product according to claim 4, kit of parts according to claim 5 or package according to claim 6 wherein such combination, product, kit of parts or package further comprises (c) another active compound selected from: (a) PDE IV inhibitors, (b) β2 agonists, (c) leukotriene D4 antagonists, (d) inhibitors of egfr-kinase, (e) p38 kinase inhibitors and (f) NK1 receptor agonists for simultaneous, separate or sequential use.

17. The combination, product, kit of parts or package according to claim 16 wherein the active compound (c) is selected from the group consisting of (a) PDE IV inhibitors and (b) β2 agonists.
